# EUROPEAN PATENT SPECIFICATION

(11) **EP 1 542 986 B1**
(45) Date of publication and mention of the grant of the patent: **24.01.2007**
(21) Application number: 02807860.8
(22) Date of filing: 24.09.2002
(51) Int. Cl.: C07D 401/04

(54) **PROCESS FOR THE PRODUCTION OF DESLORATADINE**
VERFAHREN ZUR HERSTELLUNG VON DESLORATADIN
PROCEDE POUR LA PRODUCTION DE DESLORATADINE

(43) Date of publication of application: 22.06.2005
(73) Proprietor: Morepen Laboratories Ltd., New Delhi 110 001 (IN)
(72) Inventor: Suri, Sanjay, New Delhi 110 001 (IN); Singh, Jujhar, New Delhi 110 001 (IN); Naim, Syed Shawkat, New Delhi 110 001 (IN)
(74) Representative: Goddar, Heinz J.
(86) International application number: PCT/IN2002/000193
(87) International publication number: WO 2004/029039

(56) References cited:
- EP-A- 0 270 818
- WO-A-95/10514
- WO-A-96/31478
- IWASAKI N ET AL: "AMPHOTERIC DRUGS. II. SYNTHESIS AND ANTIALLERGIC ACTIVITY OF (4-(5H-DIBENZO(A,D)CYCLOHEPTEN-5-YLIDENE)P IPERIDINO)ALKANOIC ACID DERIVATIVES AND RELATED COMPOUNDS" CHEMICAL AND PHARMACEUTICAL BULLETIN, PHARMACEUTICAL SOCIETY OF JAPAN. TOKYO, JP, vol. 42, no. II, November 1999 (1999-11), pages 2285-2290, XP000919401 ISSN: 0009-2363
- CID M M ET AL: "NEW SYNTHESIS OF CYPROHEPTADINE AND RELATED COMPOUNDS USING LOW VALENT" TETRAHEDRON, ELSEVIER SCIENCE PUBLISHERS, AMSTERDAM, NL, vol. 44, no. 19, 1988, pages 6197-6200, XP002029414 ISSN: 0040-4020

## Description

### BACKGROUND OF THE INVENTION

This invention relates to an improved process for the production of Desloratadine. The present invention particularly provides a process for the production of Desloratadine (DCL) with high yield, high purity, and very low residual solvent. Desloratadine is known as descarboethoxyloratadine. It's chemical name is 8-chloro-6,11-dihydro-11-(4-piperidilydene)-5H-benzo[5,6]cyclohepta[1,2-b] pyridine and represented by the structural formula 1.

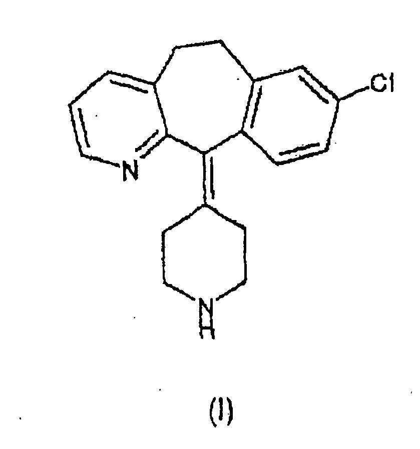

DCL is a metabolic derivative of loratadine, an H-1 histamine receptor antagonist. Loratadine has been shown to be effective in treating numerous disorders, including, but not restricting to, colds, chronic urticaria, seasonal allergic rhinitis and seasonal perennial rhinitis. Due to its antihistamic activity, it is found useful for the treatment of allergic asthma, diabetic retinopathy and other small vessel disorders associated with

the diabetes mellitus. Though loratadine belongs to a class of non-sedative antihistamines, a strong potential exists for an adverse interaction between inhibitors of cytochrome P450 and loratadine. Hence, concurrent administration of loratadine with ketoconazole, itraconazole or antibiotic like erythromycin need to be done cautiously. DCL in addition to being non-sedative antihistamine, also avoids many adverse side effects associated with loratadine. According to literature DCL is 2.5 to 4 times, more active orally than loratadine and antihistamine activity tests for 24 hrs (Arzneim. Forsch./Drug Res. 50(1) Nr.4 (345-352)2000).

Importantly, it has been shown that DCL is 5 to 7 times less active in tumor promotion than loratadine and is at least 20 times more potent at histamine receptor when compared to loratadine. Hence it is more desirable to have DCL containing pharmaceutical compositions. However, over time DCL with lactose produce a brown coloured product and there is a high degree of DCL degradation.

The Hungarian Patent Number 194864, describes following two methods for the preparation of desloratadine from Loratadine (chemical name: 8-chloro-6, 11-dihydro-11-(1-ethoxy carbonyl-4-piperidilydene) -5H-benzo [5,6] cyclohepta[1,2-b] pyridine:
(a) the 8-chloro-6, 11-dihydro-11-(1-ethoxycarbonyl-4-piperidilydene) -5H-benzo [5,6] cyclohepta[1,2-b] pyridine (loratadine) is decarbethoxylated by boiling with aqueous ethanolic sodium hydroxide solution for 24 hrs, then isolating DCL as acetate after neutralizing the solution with acetic acid. The crude product has to be further purified. The yield after recrystallization from benzene-hexane mixture amounts to 70%
(b) 8-chloro-6, 11-dihydro-11-(1-methyl-4-piperidilydene)-5H-benzo [5,6] cyclohepta[1,2-b] pyridine (azatadine) is demethylated in 2 steps: the 1-cyano derivative is synthesized with cyanogen bromide and then hydrolysed with concentrated HCl in acetic acid for 20 hrs. The residue after evaporating solvent is neutralized with NH₄OH solution to get DCL.

The above processes have several disadvantages. During the realization of process substantial decomposition takes place leaving several impurities in the final product. While purifying the product by recrystallisation, substantial material is lost. The base obtained is insoluble in water and poses problems in the preparation of formulation. Process (b) is disadvantageous in itself, because of the use of poisonous cyanogen bromide reagent and the poisonous methyl bromide formed. It also had the disadvantageous of the method (a).

The US Patent No. 4, 659, 716 also teaches preparation of DCL by dealkylation of azatadine by reaction with cyanogen bromide followed by acid hydrolysis and decarbethoxylation of loratadine by refluxing either with KOH and EtOH/Water (1:1) for 66 hrs or NaOH and 70% EtOH for 24 hrs with yield ranging from 90 to 95%. The product obtained is coloured in addition to the disadvantages outlined above and thus needs further purification.

US Patent No. 4, 826, 853 exemplifies the preparation of DCL by cleaving ethoxy group of loratadine via acid (HCl) or base (KOH) hydrolysis. It also advises treating it with organometallic reagent (CH₃Li) and a reductive agent (Zn in acid). DCL is also prepared by refluxing Loratadine with KOH in aqueous ethanol for 64 hrs. Recrystallisation can be carried out using toluene. The yield is 77%.

As per US Patent No. 5, 925, 648 and PCT No. WO98/11,092 and WO98/04545 (US.97/12923), DCL may be prepared by hydrolyzing loratadine as specified above wherein refluxing may be effected for about 60 hrs using EtOH and HCl in anhydrous state followed by basifying with KOH or NaOH and extracting with CH₂Cl₂.

European Patent No. 0208855 also teaches the preparation of DCL by following methods:
- dealkylation of azatadine using cyanogen bromide followed by hydrolysis; and
- refluxing Loratadine with KOH and aqueous ethanol for 66 hrs to give 95% yield as acetate. A combination of EtOH/Water (1:1) and NaOH also has been exemplified.

PCT application No. WO 8503707 or European Patent No. 0152897 also teaches preparation of DCL either by demethylation through cyanogen route or through decarbethoxylation by refluxing loratadine with EtOH and NaOH for 24 hrs resulting into crude coloured product as thick emulsion that can not be filtered or isolated easily. More over, since the product obtained is crude, requires further purification. 6.0 g of loratadine results in to 4.0 to 4.5 g of DCL European Patent application No. 93108177.2 [0577957A1 (ES2, 042, 421)] teaches preparation of DCL by treating loratadine with dry chloroform and trimethylsilyl iodide at 55 to 60°C overnight followed by treatment with HCl and basifying with NaOH. Product needs extraction and further purification. The yield is 77%.

PCT application No. WO96/31478 proposes preparation of iodoamine through hydrolysis procedure prescribed in Example 358 step A of WO 95/10516 with 89% yield and preparation of bromoamine through again hydrolysis prescribed in Example 358 step A of WO 95/10516 with 69% yield. This procedure may be applicable to the preparation of DCL.

PCT application No. WO 95/10514 also teaches preparation of DCL via cyanogen route or alkali hydrolysis route. It exemplifies use of aqueous Ethanol and KOH giving 77% yield after reflux for 64 hrs.

US Patent No. 5, 595, 997 advocates saponification of loratadine using sodium hydroxide and absolute ethanol to produce DCL after reflux for 4 days as pale-tan solid. The derivative needs to be extracted with methylene chloride.

PCT application No. WO 95 10516 suggests preparation applicable to DCL via acid hydrolysis of loratadine using HCl followed by neutralizing with NaOH and extracting with CH₂Cl₂.

PCT application No. WO 99/01450, basically teaching the preparation of DCL polymorphs 1 & 2 advises preparation of DCL polymorph 1 through reflux of loratadine with KOH flakes in industrial methylated spirit for 3 hrs followed by addition of water and crystallizing with MIBK.

Reference also can be made to PCT application No. WO / 03707, WO 92/00293, WO 95/10515 that describes the preparation of DCL.
It can be seen that the existing processes either relate to treating azatadine with cyanogen bromide followed by acid hydrolysis & neutralization or relate to alkali or acid hydrolysis of loratadine using alkali metal hydroxide with aqueous alcohol. The processes involve refluxing for a longer period ranging from 24 to 66 hrs or even more upto 4 days. The product is invariably coloured and needs further purification.
The yield varies from 77 to 90 %. The processes suffer from one or the other disadvantages as outlined above.

### SUMMARY OF THE INVENTION

In our experiments, we have found out that the process of the present invention is unexpectedly advantageous for the commercial scale production of the title compound with high yield, high purity, and low value residual solvent The process is more economic in addition to being eco-friendly. It has greatest degree of reproducibility.

Accordingly the main object of the present invention is to provide a process for the production of Desloratadine having structural formula 1 which obviates the draw backs of the existing processes.

Another object is to provide a process viable for the commercial production of the title compound.

Still another object of the present invention is to provide a process that can produce a compound, which meets. GMP requirements, ICH requirements and health registration requirements Further the compound should be in pure form having consistent properties with minimum side effects and good stability.

Accordingly the present invention provides process for the production of Desloratadine according to claim 1. Preferred embodiments are disclosed in the subclaims.

One of the embodiments of the present invention is that the alcohol used may be alkanols of 1 to 10 carbon atoms.
The alkanols of 1 to 10 carbon atoms used may be methanol, ethanol, propanol, isopropanol, tert. butyl alcohol, pentanol, hexanol, cycloalkanols such as cyclohexanol; aromatic alcohols such as benzyl alcohol.
However, the scope of the present invention is not limited to the above mentioned alcohols, but can also be extended to other alcohols. The alcohol used may preferably be a C₁-C₄ alkanol, more preferably methanol.

The another embodiment is that the amount of alcohol may vary between 1 and 10 w/v equivalents calculated on the starting compound- loratadine.

The amount of alcohol used may preferably be 2-6 equivalents, more preferably be 4 equivalents.

Yet another embodiment of the invention is that the inorganic base which may be used in the process of the invention are alkali metal hydroxide.
The alkali metal hydroxide used may be such as sodium hydroxide, potassium hydroxide The preferred alkali metal hydroxide used, may be sodium hydroxide.
The amount of base used may vary between 0.5 and 1.6 w/w equivalents calculated on the starting compound - loratadine.
Preferably 1-1.6 equivalents of base may be used, more preferably 1.1 equivalents may be used.

The reaction may be carried out at a temperature between 60° and 100° C or at respective refluxing temperature, preferably between 80° and 95° C more preferably 85 to 90 °C.

The amount of water used for crystallization may be at least 2 times of the solvent employed. Preferable amount of water needed may be four times of the solvent used.

### DETAILED DESCRIPTION OF THE INVENTION

Among the various processes known in the art, carbethoxylation of loratadine appears to be more relevant. The processes disclosed in the Hungarian Patent Number 194 864, European Patent No.0152 897 and PCT application NO.WO 8503707, teach boiling/refluxing loratadine with Aqueous ethanol and KOH for 24 hrs., which results in the production crude form of DCL with about 70% yield. US Patent No. 5,595,997 advocates saponification of Loratadine using NaOH and absolute EtOH for 4 days to produce DCL as pale tan solid. US Patent No. 4,659,716;4,826,853 also advise refluxing Loratadine with Aqueous EtOH and KOH for about 60 hrs to produce DCL.
The literature does not report use of neat alcohol in combination with alkali metal hydroxide to produce stable DCL from loratadine.
In the process according to this invention, loratadine is reacted with neat alcoholin presence of an inorganic base to produce DCL.

According to a preferred realization loratadine is heated at a temperature between 60°C to 100°C or at respective refluxing temperature.

Further, it is observed that the loratadine when heated with neat MeOH and NaOH for about 1 to 6 hrs produces DCL that can be isolated easily by adding excess of water in pure crystalline form with high yield. The product obtained does not develop any colour on storage and meets all ICH requirements, GMP requirements & health registration requirements. Thus the process of the present invention is easy to operate, environment friendly economic and useful for commercial production.
The starting material of the compound of the invention is loratadine (8-chloro-6,11-dihydro-11-(1-ethoxycarbonyl-4-piperidilydene(-5H-benzo[5,6]cyclohepta [1,2b]pyridine). The synthesis of loratadine is described in detail in the US Patent no. 4,282,233.

During the course of the development of the process of the present invention, DCL was found to develop colour on storing for about > 4 months when KOH is used as inorganic base. Further additional efforts such as purification with MeOH-isopropyl ether, are needed to get the compound of required quality.

It has been observed that the increase is directly proportional to reaction time and decrease in the base results in increase in the reaction time.

It has also been noticed that inorganic base such as Lithium compounds and carbonates of alkali metals do not work. Similarly polyols fail to produce DCL.

The process of the present invention is further illustrated by the following examples. However, it should not limit the scope of the invention.

### EXAMPLE - 1

A mixture of 8-chloro-6, 11-dihydro-11-(1-ethoxycarbonyl-4-piperidilydene) - 5H- benzo [5,6] cyclohepta [1,2-b] pyridine (100 gm, 0.2612 mole), sodium hydroxide (110 gm, 2.75 mole) in methanol (400 ml, 9.89 mole) was refluxed for 2 hrs at 82o to 95oC. After completion of reaction, 1000 ml of water is added to obtain crystals of 8-chloro-6, 11-dihydro-11-(4-piperidilydene) - 5H-benzo [5,6] cyclohepta [1,2-b] pyridine, which was filtered at 25o-30oC, washed with plenty of water to remove salts, dried at 50-55oC. The isolated yield was 76 gm., with a purity of 99.8% (OAB, HPLC) and an absorbance of 0.043 at 430 nm. giving a yield of 93.6%

The structure of the compound was confirmed by comparison of its I.R., NMR and Mass Spectra of reference standards. The quality results are shown in Table - 3.

### EXAAIPLE - 2

The reaction is carried out as described in Example 1, but the amount of methanol used was 600ml (14.83 mole) instead of 400ml. The reaction was completed in 12 hrs. The isolated yield of 8-chloro-6, 11-dihydro-11-(4-piperidilydene) - 5H-benzo [5,6] cyclohepta (1,2-b) pyridine was 72 gm with a purity of 99.6% (OAB, HPLC) and an absorbance of 0.083 at 430 nm. giving a yield of 88.7%

### EXAMPLE - 3

The reaction was carried out as described in Example 1, but the amount of sodium hydroxide used was 92 gm (2.3 mole) instead of 110 gm. The reaction is completed in 8 hrs. The isolated yield of 8-chloro-6, 11-dihydro-11-(4-piperidilydene) - 5H-benzo [5,6] cyclohepta (1,2-b) pyridine was 71 gm with a purity of 99.7% (OAB, HPLC) and an absorbance of 0.09 at 430 nm. giving a yield of 87.5%

### EXAMPLE - 4

The reaction was carried out as described in Example 1, but the ethanol (400 ml, 6.83 mole) was used instead of methanol (400ml). The reaction is completed in 5 hrs. The isolated yield of 8-chloro-6, 11-dihydro-11-(4-piperidilydene) - 5H-benzo [5,6] cyclohepta (1,2-b) pyridine was 72 gm with a purity of 99.5% (OAB, HPLC) and an absorbance of 0.6 at 430 nm. giving a yield of 88.7% Further purification with methanol- isopropyl ether results in 92% yield, and 0.1 absorbance.

### EXAMPLE - 5

The reaction was carried out as described in Example 1, but the isopropanol (400ml, 5.23 mole) and potassium hydroxide (160gm, 2.857 mole) were used instead of methanol (400ml) and sodium hydroxide (110gm). The reaction is completed in 4 hrs. The isolated yield of 8-chloro-6, 11-dihydro-11-(4-piperidilydene) - 5H-benzo [5,6] cyclohepta (1,2-b) pyridine was 75 gm with a purity of 99.6% (OAB, HPLC) and an absorbance of 0.27 at 430 nm. giving a yield of 92.4% further purification as in example 4 results in the compound with absorbance of 0.07. The yield has increased to 83.8%

### EXAMPLE - 6

The reaction was carried out as described in Example 5, but the amount of isopropanol and potassium hydroxide used was one litre (21.23 mole) and 100gm (1.786 mole) respectively instead of 400ml and 160 gm. The reaction is completed in 50 hrs. The isolated yield of 8-chloro-6, 11-dihydro-11-(4-piperidilydene) - 5H-benzo [5,6] cyclohepta (1,2-b) pyridine was 76 gm with a purity of 99.7% (OAB, HPLC) and an absorbance of 0.2 at 430 nm. giving a yield of 93.62% On purification yield was 82.5 and colour absorbance was 0.06.

### EXAMPLE - 7

The reaction was carried out as described in Example 1, but the n-propanol (400ml, 5.36 mole) was used instead of methanol (400ml). The reaction is completed in 4 hrs. The isolated yield of 8-chloro-6, 11-dihydro-11-(4-piperidilydene) - 5H-benzo [5,6] cyclohepta (1,2-b) pyridine was 72 gm with a purity of 99.5% (OAB, HPLC) giving a yield of 88.7%. Further purification in methanol- isopropyl ether provides the yield of 64 gm (78.8 %) with an absorbance of 0.09 (430 nm).

### EXAMPLE - 8

The reaction was carried out as described in Example 1, but the n-propanol (400ml, 5.36 mole) and potassium hydroxide (160gm, 2.857 mole) were used instead of methanol (400ml) and sodium hydroxide (110gm). The reaction is completed in one hrs. The isolated yield of 8-chloro-6, 11-dihydro-11-(4-piperidilydene) - 5H-benzo [5,6] cyclohepta (1,2-b) pyridine was 74 gm with a purity of 99.47% giving a yield of 91.2%. Further purification in methanol- isopropyl ether was achieved to get a yield of 66gm (81.3%) with an absorbance of 0.08 (430 nm).

### EXAMPLE - 9

The reaction was carried out as described in Example 1, but the n-butanol (400ml, 4.37 mole) was used instead of methanol (400ml) The reaction is completed in 3 hrs. The isolated yield of 8-chloro-6, 11-dihydro-11-(4-piperidilydene) - 5H-benzo [5,6] cyclohepta (1,2-b) pyridine was 72 gm with a purity of 99.6% (OAB, HPLC) giving a yield of 88.7%. This was further purified to get an absorbance of 0.08 and yield of 65 gm (80.1%).

### EXAMPLE - 10

The reaction was carried out as described in Example 1, but the n-butanol (400ml, 4.37 mole) and potassium hydroxide (160gm, 2.857 mole) were used instead of methanol (400ml) and sodium hydroxide (110gm). The reaction is completed in one hrs. The isolated yield of 8-chloro-6, 11-dihydro-11-(4-piperidilydene) - 5H-benzo [5,6] cyclohepta (1,2-b) pyridine was 74 gm which is further purified in methanol- isopropyl to get product with a purity of 99.7% (OAB, HPLC) and an absorbance of 0.08 (430 nm) giving a yield of 82.5%.

### EXAMPLE - 11

The reaction was carried out as described in Example 1, but the benzyl alcohol (400ml, 3.87 mole) was used instead of methanol (400ml). The reaction is completed in 3 hrs. The isolated yield of 8-chloro-6, 11-dihydro-11-(4-piperidilydene) - 5H-benzo [5,6] cyclohepta (1,2-b) pyridine was 73 gm which was further purified in methanol- isopropyl ether to get purity 99.76% (OAB, HPLC) and an absorbance of 0.08 (430 nm) giving a yield of 80.1%.

### EXAMPLE - 12

The reaction was carried out as described in Example 1, but the benzyl alcohol (400ml, 3.87 mole) and potassium hydroxide (160gm, 2.857 mole) were used instead of methanol (400ml) and sodium hydroxide (110gm). The reaction is completed in 1.5 hrs. The isolated yield of 8-chloro-6, 11-dihydro-11-(4-piperidilydene) - 5H-benzo [5,6] cyclohepta (1,2-b) pyridine was 74 gm as base. This was crystallized in methanol- isopropyl ether to get a purity of 99.6% (OAB, HPLC) and an absorbance of 0.09 (430 nm) giving a yield of 66gm (81.3%).

Table 1 & Table 2 are hereby incorporated to indicate reaction time and quality results of preparation of DCL and proportion of various ingredients used for the preparation of DCL, respectively.

**Table -1 Reaction time and quality result of preparation of 8-chloro-6, 11-dihydro-11- (-4-piperidylidene) - 5H- benzo [5,6] cyclohepta [1,2-b] pyridine (Formula -1).**

| **S. No.** | **Lora tdine (gm)** | **Neat Solvents (ml)** | | | | | | | | **Base (gm)** | | **Purity (HPLC ) (%)** | **Yield (%)** | **Colour Absorbance at 430 nm** | **Reaction Time (hrs)** | **Remarks** |
|---|---|---|---|---|---|---|---|---|---|---|---|---|---|---|---|---|
| | | **MeO H** | **EtOH** | **n-Pr.** | **n- But.** | **i-Pr.** | **Benzyl Alco hol** | **DM SO** | | **NaoH** | **KoH** | | | | | |
| **01.** | 100 | 400 | - | - | - | - | - | - | | 110 | - | 99.8 | 93.6 | 0.043 | 2 | NP |
| **02.** | 100 | 600 | - | - | - | - | - | - | | 110 | - | 99.6 | 88.7 | 0.083 | 12 | " |
| **03.** | 100 | 400 | - | - | - | - | - | - | | 92 | - | 99.7 | 87.5 | 0.09 | 8 | " |
| **04.** | 100 | 400 | - | - | - | - | - | - | | - | 160 | 99.7 | 92.0 | 0.10 | 3 | " |
| **05.** | 100 | - | 400 | - | - | - | - | - | | 110 | - | 99.5 | 77.6 | 0.10 | 5 | P |
| **06.** | 100 | - | 400 | - | - | - | - | - | | - | 160 | 99.6 | 78.0 | 0.12 | 2 | " |
| **07.** | 100 | - | - | 400 | - | - | - | - | | 110 | - | 99.5 | 78.8 | 0.09 | 4 | " |
| **08.** | 100 | - | - | 400 | - | - | - | - | | - | 160 | 99.4 | 81.3 | 0.08 | 1 | " |
| **09.** | 100 | - | - | - | 400 | - | - | - | | 110 | - | 99.6 | 80.1 | 0.08 | 3 | " |
| **10.** | 100 | - | - | - | 400 | - | - | - | | - | 160 | 99.7 | 82.5 | 0.08 | 1 | " |
| **11.** | 100 | - | - | - | - | 400 | - | - | | - | 160 | 99.7 | 83.8 | 0.07 | 4 | " |
| **12.** | 100 | - | - | - | - | 300 | - | - | | - | 75 | 99.6 | 84.2 | 0.07 | 21 | " |
| **13.** | 100 | - | - | - | - | 500 | - | - | | - | 100 | 99.5 | 85.0 | 0.09 | 20 | " |
| **14.** | 100 | - | - | - | - | 1000 | - | - | | - | 100 | 99.7 | 82.5 | 0.06 | 50 | " |
| **15.** | 100 | - | - | - | - | - | 400 | - | | 110 | - | 99.7 | 80.1 | 0.08 | 3 | " |
| **16.** | 100 | - | - | - | - | - | 400 | - | | - | 160 | 99.6 | 81.3 | 0.09 | 1.5 | " |
| **17.** | 100 | - | - | - | - | - | - | 400 | | 110 | - | 99.4 | 80.3 | 0.12 | 3 | " |
| **18.** | 100 | - | - | - | - | - | - | 400 | | - | 160 | 99.5 | 80.1 | 0.15 | 3 | " |
| **19.** | 100 | 300 | - | - | - | - | - | - | | - | 75 | 99.6 | 92.1 | 0.09 | 30 | NP |
| **20.** | 100 | 300 | - | - | - | - | - | - | | - | 100 | 99.7 | 92.8 | 0.08 | 20 | " |

| | | | | | | | | | | | | | | | | |
|---|---|---|---|---|---|---|---|---|---|---|---|---|---|---|---|---|
| NP- No Purification Required P- Purification Required | | | | | | | | | | | | | | | | |

**Table - 2 Reaction for the preparation of 8-chloro-6, 11-dihydro-11- (4-piperidylidene) - 5H- benzo [5,6] cyclohepta [1,2-b] pyridine (Formula-1) were failed in the following combination of Solvents and Base.**

| **S. No.** | **Loratadine (gm)** | **Neat Solvents (ml)** | | | | | **Base (gm)** | | | | **Reaction** |
|---|---|---|---|---|---|---|---|---|---|---|---|
| | | **MeOH** | **Ethylon Glycol** | **1,2,3- Propane Triol** | **DMF** | | **NaoH** | **KoH** | **Na₂CO₃** | **K₂CO₃** | |
| **01.** | 100 | 400 | - | - | - | | - | - | 124 | - | No reaction |
| **02.** | 100 | 400 | - | - | - | | - | - | - | 190 | " |
| **03.** | 100 | - | - | - | 400 | | 110 | - | - | - | " |
| **04.** | 100 | - | - | - | 400 | | - | 160 | - | - | " |
| **05.** | 100 | - | 400 | - | - | | 110 | - | - | - | " |
| **06.** | 100 | - | 400 | - | - | | - | 160 | - | - | " |
| **07.** | 100 | - | - | 400 | - | | 110 | - | - | - | " |
| **08.** | 100 | - | - | 400 | - | | - | 160 | - | - | " |

**Table - 3 Quality Result of 8-chloro-6, 11-dihydro-11- (4-piperidylidene) - 5H- benzo [5,6] cyclohepta [1,2-b] pyridine described in Example -1 (three batches)**

| S. **NO.** | **TESTS** | **EXAMPLE-1** | | |
|---|---|---|---|---|
| | | **Ia** | **Ib** | **Ic** |
| **01.** | Water (% w/w) | 0.25 | 0.20 | 0.27 |
| **02.** | Colour Absorbance (A) at 430 nm | 0.048 | 0.050 | 0.043 |
| **03.** | Melting Range (°C) | 152-155 | 152-155 | 152-155 |
| **04.** | Assay (OAB, HPLC) % w/w | 99.72 | 99.78 | 99.85 |
| **05.** | Impurities (% w/w) total | 0.06 | 0.06 | 0.06 |
| **06.** | Sulphated Ash (% w/w) | 0.03 | 0.05 | 0.05 |
| **07.** | Residual Solvents (Methanol) ppm | 1167 | 762 | 1933 |

## Claims

1. Process for the production of Desloratadine which comprises reacting loratadine with neat alcohol in presence of inorganic base, precipitating the title compound in crystalline form by addition of excess water and isolating the crystals obtained.

2. Process as claimed in claim 1 wherein the alcohol used is alkanols of 1 to 10 carbon atoms.

3. Process as claimed in claim 2 wherein the alkanols of 1 to 10 carbon atoms used are methanol, ethanol, propanol, isopropanol, tert. butyl alcohol, pentanol, hexanol, cycloalkanols such as cyclohexanol; aromatic alcohols such as benzyl alcohol.

4. Process as claimed in claim 1 wherein the alcohol used is a C₁-C₄ alkanol, preferably methanol.

5. Process as claimed in claim 1 wherein the amount of alcohol used vary between 1 and 10 (w/v) equivalents calculated on the starting compound loratadine.

6. Process as claimed in claim 1 wherein the amount of alcohol used is 2-6 (w/v) equivalents, preferably be 4 equivalents.

7. Process as claimed in claim 1 wherein the inorganic base used is alkali metal hydroxides.

8. Process as claimed in claim 7 wherein the alkali metal hydroxide such as sodium hydroxide, potassium hydroxide are used.

9. Process as claimed in claim 7 wherein the alkali metal hydroxides used is sodium hydroxide.

10. Process as claimed in claim 1 wherein the amount of inorganic base used vary between 0.5 and 1.6 (w/w) equivalents calculated on the starting compound loratadine.

11. Process as claimed in claim 1 wherein 1-1.6 (w/w) equivalents of base is used.

12. Process as claimed in claim 1 wherein the base used is 1.1 (w/w) equivalents.

13. Process as claimed in claim 1 wherein the reaction is carried out at a temperature between 60° and 100°C or at respective refluxing temperature, preferably between 80° and 95°C more preferably between 85 to 90°C.

14. Process as claimed in claim 1 wherein the amount of water added is 2 to 4 times of the solvent employed.

15. Process as claimed in claim 1 wherein the isolation is effected by filtration.

## Patentansprüche

1. Verfahren für die Herstellung von Desloratadin, welches ein Umsetzen von Loratadin mit reinem Alkohol in der Gegenwart von anorganischer Base, Ausfällen der Titelverbindung in kristalliner Form durch Zugabe von Wasser im Überschuß und Isolieren der erhaltenen Kristalle umfaßt.

2. Verfahren nach Anspruch 1, wobei der verwendete Alkohol Alkanole von 1 bis 10 Kohlenstoffatomen ist.

3. Verfahren nach Anspruch 2, wobei die verwendeten Alkanole von 1 bis 10 Kohlenstoffatomen Methanol, Ethanol, Propanol, Isopropanol, tert.-Butylalkohol, Pentanol, Hexanol, Cycloalkanole, wie Cyclohexanol; aromatische Alkohole, wie Benzylalkohol, sind.

4. Verfahren nach Anspruch 1, wobei der verwendete Alkohol ein C₁-C₄-Alkanol, bevorzugt Methanol ist.

5. Verfahren nach Anspruch 1, wobei die Menge an verwendetem Alkohol zwischen 1 und 10 (w/v) Äquivalenten, berechnet auf der Ausgangsverbindung Loratadin, variiert.

6. Verfahren nach Anspruch 1, wobei die Menge an verwendetem Alkohol 2-6 (w/v) Äquivalente, bevorzugt 4 Äquivalente, ist.

7. Verfahren nach Anspruch 1, wobei die anorganische Base Alkalimetallhydroxid ist.

8. Verfahren nach Anspruch 1, wobei das Alkalimetallhydroxid, wie Natriumhydroxid, Kaliumhydroxid, verwendet wird.

9. Verfahren nach Anspruch 7, wobei das verwendete Alkalimetallhydroxid Natriumhydroxid ist.

10. Verfahren nach Anspruch 1, wobei die Menge an verwendeter anorganischer Base zwischen 0,5 und 1,6 (w/w) Äquivalenten, berechnet auf der Ausgangsverbindung Loratadin, variiert.

11. Verfahren nach Anspruch 1, wobei 1-1,6 (w/w) Äquivalente als Base verwendet werden.

12. Verfahren nach Anspruch 1, wobei die verwendete Base 1,1 (w/w) Äquivalente ist.

13. Verfahren nach Anspruch 1, wobei die Reaktion bei einer Temperatur zwischen 60° und 100°C oder bei einer entsprechenden Rückflußtemperatur, bevorzugt zwischen 80° und 95°C, bevorzugter zwischen 85 und 90°C durchgeführt wird.

14. Verfahren nach Anspruch 1, wobei die Menge an zugegebenem Wasser 2- bis 4-mal die Menge des eingesetzten Lösungsmittels ist.

15. Verfahren nach Anspruch 1, wobei die Isolierung durch Filtration bewirkt wird.

## Revendications

1. Procédé pour la production de Desloratadine comprenant la réaction de la loratadine avec de l'alcool pur en présence d'une base inorganique, consistant en la précipitation du composé du titre sous forme cristalline par l'addition d'un excédant d'eau et l'isolation des cristaux obtenus.

2. Procédé tel que décrit dans la revendication 1, selon lequel l'alcool utilisé consiste en alcanols de 1 à 10 atomes de carbone.

3. Procédé tel que décrit dans la revendication 2, selon lequel les alcanols de 1 à 10 atomes de carbone utilisés sont du méthanol, de l'éthanol, du propanol, de l'isopropanol, du tert, de l'alcool butylique, du pentanol, de l'hexanol, des cycloalcanols tels que le cyclohexanol ; des alcools aromatiques tels que l'alcool benzylique.

4. Procédé tel que décrit dans la revendication 1, selon lequel l'alcool utilisé est de l'alcanol C₁-C₄, de préférence du méthanol.

5. Procédé tel que décrit dans la revendication 1, selon lequel la quantité d'alcool utilisée varie entre 1 et 10 équivalents (p/v) calculés en fonction du composé de départ de loratadine.

6. Procédé tel que décrit dans la revendication 1, selon lequel la quantité d'alcool utilisée est de 2-6 équivalents (p/v), de préférence de 4 équivalents.

7. Procédé tel que décrit dans la revendication 1, selon lequel la base inorganique utilisée consiste en hydroxydes métalliques alcalins.

8. Procédé tel que décrit dans la revendication 7, selon lequel l'hydroxyde métallique alcalin utilisé consiste en hydroxydes tels que l'hydroxyde de sodium, l'hydroxyde de potassium.

9. Procédé tel que décrit dans la revendication 7, selon lequel les hydroxydes métalliques alcalins utilisés consistent en hydroxyde de sodium.

10. Procédé tel que décrit dans la revendication 1, selon lequel la quantité de base inorganique utilisée varie entre 0,5 et 1,6 équivalents (p/p) calculés en fonction du composé de départ de loratadine.

11. Procédé tel que décrit dans la revendication 1, selon lequel 1 à 1,6 équivalents (p/p) de la base est utilisé.

12. Procédé tel que décrit dans la revendication 1, selon lequel la base utilisée consiste en 1,1 équivalents (p/p).

13. Procédé tel que décrit dans la revendication 1, selon lequel la réaction est réalisée à une température de 60° à 100°C ou à une température de refluxage respective, de préférence de 80° à 95°C, idéalement de 85 à 90°C.

14. Procédé tel que décrit dans la revendication 1, selon lequel la quantité d'eau ajoutée est de 2 à 4 fois celle du solvant employé.

15. Procédé tel que décrit dans la revendication 1, selon lequel l'isolement est réalisé par filtration.
